(19) Europäisches Patentamt
European Patent Office
Office européen des brevets

(11) Veröffentlichungsnummer: **0 302 816 B1**

(12) **EUROPÄISCHE PATENTSCHRIFT**

(45) Veröffentlichungstag der Patentschrift: **26.08.92**

(51) Int. Cl.5: **C11B 9/00**, A61K 7/46

(21) Anmeldenummer: **88730164.6**

(22) Anmeldetag: **21.07.88**

# (54) Verwendung von Campholennitrilen als Riechstoff.

(30) Priorität: **07.08.87 DE 3726418**

(43) Veröffentlichungstag der Anmeldung:
**08.02.89 Patentblatt 89/06**

(45) Bekanntmachung des Hinweises auf die Patenterteilung:
**26.08.92 Patentblatt 92/35**

(84) Benannte Vertragsstaaten:
**AT CH DE ES FR GB IT LI NL**

(56) Entgegenhaltungen:
**GB-A- 2 053 199**

**CHEMICAL ABSTRACTS BAND 77, Nr. 7, 14. August 1972, Seite 509; Spalte 1; Zusammenfassung Nr. 48644; G. PIRISINO: "Action of Alkylmagnesium halides on alpha and beta campholenonitriles and the corresponding amides";& ANN. CHIM. (ROME) 1972, Band 62, Nr. 2, Seiten 113-127**

(73) Patentinhaber: **Dragoco Gerberding & Co. GmbH**
**Dragocostrasse 1**
**W-3450 Holzminden(DE)**

(72) Erfinder: **Brunke, Ernst-Joachim, Dr. Dipl.-Chem.**
**Pippingsbusch 3**
**W-3450 Holzminden(DE)**
Erfinder: **Kappey, Claus-Hermann, Dr. Dipl.-Chem.**
**Schratweg 1**
**W-3450 Holzminden(DE)**

(74) Vertreter: **Eikenberg, Kurt-Rudolf**
**Patenanwalt Schackstrasse 1**
**W-3000 Hannover 1(DE)**

EP 0 302 816 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS Band 102, Nr. 1, 7, Januar 1985, Seite 715; Spalte 1; Zusammenfassung Nr. 24837; N.G. KOZLOV et al.: "Terpene amines. Secondary amines based on beta campholenic acid nitrile"; & ZH. ORG. KHIM. (RUSS.), 1984, Band 20. Nr. 7, Seiten 1417-1421

CHEMICAL ABSTRACTS Band 101, Nr. 10, 3. September 1984, Seite 669; Spalte 2, Zusammenfassung Nr. 91245; I.I. BARDYSHEV et al.: "Hydroamination of ketones by alpha-campholenic acid nitrile"

CHEMICAL ABSTRACTS Band 96, Nr. 13, 29. März 1982, Seite 651; Spalte 1;Zusammenfassung Nr. 122287 & PL. 110052; C. WAWRZENCZYK et al.: "Trimethylcyclopentene derivative" 30-04-1981

**Beschreibung**

Verbindungen mit Campholen-Grundgerüst kommen in der Natur als Bestandteile etherischer Öle vor, z.B. α-Campholenaldehyd 2 in Wacholderbeeröl (A.F. Thomas, Helv. Chim. Acta, 55, 815 (1972). α-Campholenaldehyd besitzt einen frisch-harzigen Geruch.

Einige Derivate des Campholenaldehyds 2 mit verlängerter Seitenkette besitzen Geruchsnoten holzig-animalischen Typs, die bestimmten Noten des ostindischen Sandelholzöles entsprechen (Übersicht in: E.-J. Brunke und E. Klein "Chemistry of Sandalwood Fragrance", in "Fragrance Chemistry" (Editor E. Theimer), Academic Press, New York 1982, S. 424 - 426)).

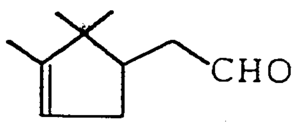

2

Generell besteht ein ständiger Bedarf an synthetischen Riechstoffen, die sich günstig und mit gleichbleibender Qualität herstellen lassen, bei längerer Lagerung auch im Kontakt mit anderen Stoffen stabil bleiben und erwünschte olfaktorische Eigenschaften haben, d.h. angenehme, möglichst naturnahe Duftnoten von ausreichender Intensität und in der Lage sind, den Duft von kosmetischen oder technischen Verbrauchsgütern vorteilhaft zu beeinflussen. Es wurde nun gefunden, daß α-Campholennitril der allgemeinen Formel **A** und γ-Campholennitril der allgemeinen Formel **B** diese Forderung ganz ausgezeichnet erfüllen, weil sie problemlos herstellbare, außerordentlich stabile Verbindungen mit sehr individueller Geruchscharakteristik und starker Ausstrahlung sind, die vielseitig verwendbare Riechstoffe darstellen. Sie können aufgrund ihrer geruchlichen Besonderheiten zur Bereicherung der kreativen Parfümerie beitragen, und zwar als Bestandteile von Parfümölen der unterschiedlichsten Geruchsrichtungen. Durch ihre

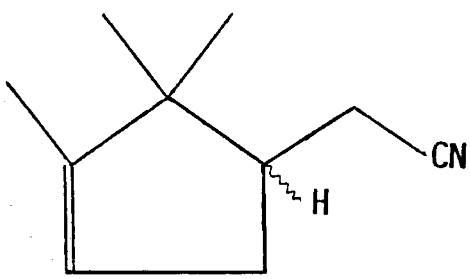

A

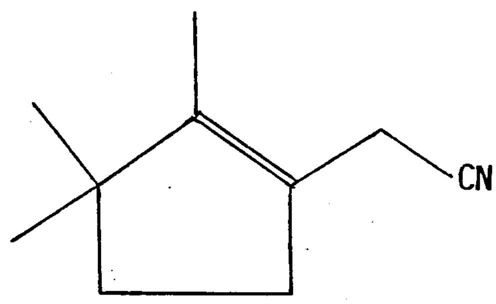

B

hohe chemische Stabilität, insbesondere Säurebeständigkeit, lassen sie sich vorteilhaft auch zur Parfümierung relativ aggressiver Grundmassen (Waschmittel, Haushaltsreiniger usw.) einsetzen.

Demgemäß bezieht sich die Erfindung auf die Verwendung von α-Campholennitril mit der allgemeinen Formel **A** (nachfolgend auch mit **4** bezeichnet) sowie von γ-Campholennitril mit der allgemeinen Formel **B** - (nachfolgend auch mit 6 bezeichnet) als Riechstoff oder als Bestandteil von Riechstoffmischungen zur Parfumierung von kosmetischen oder technischen Verbrauchsgütern. Die allgemeine Formel **A** umfasst dabei auch die stereoisomeren Formen, wie sie durch die geschlängelte Linie angedeutet sind. Beide Nitrile **A** und **B** können in reiner Form oder als Gemisch erfindungsgemäß verwendet werden, wobei das Gemisch vorzugsweise einen Gehalt von 0,5 - 99,5% an Nitril **A** bzw. 99,5 - 0,5% an Nitril **B** aufweist.

Das dem Campholenaldehyd 2 entsprechende Nitril **A** ist bereits im Jahr 1883 von V. Meyer durch Einwirkung von Methylchlorid auf Campher-oxim erhalten worden; seinerzeit war die Sturktur des Produktes aber noch nicht bekannt (V. Meyer, Ber. Dt. Chem. Ges. 16, 2981 (1883)). F. Tiemann gelang die Strukturaufklärung und Zuordnung der Formel **A** (F. Tiemann, Ber. Dt. Chem. Ges. 29, 3006 (1896)); in der gleichen Arbeit beschreibt der Autor die Herstellung von **A** durch Einwirkung von verdünnter Schwefelsäure

auf Campher-oxim. Pyrolyse oder Photolyse von Campher-oxim ergeben u.a. ebenfalls das Nitril **A** (T. Sato und H. Obase, Tetrahedron Lett. 1967, 1633-36). Weiterhin wurde **A** aus Campher-oxim auch durch Einwirkung von Phosphorpentoxid (M. Nazir et al., Pakistan J. Sci. Ind. Res. 10(1) 13-16 (1967) oder konzentrierte Salzsäure erhalten (N.G. Kozlov und T. Pekh, Zh. Org. Khim. 1982, 18(5), 1118-9)). Im Reaktionsgemisch der Photolyse oder Pyrolyse von Campher-nitrimin lag ebenfalls **A** vor (L.J. Winters, J.F. Fisher und E.R. Ryan, Tetrahedron Letters 1971, 129-132). Auch das Nitril **B** ist in der Literatur beschrieben, und zwar als Ausgangsprodukt zur Herstellung entsprechender Amine (N.G Kozlov, A.S. Zhavrid und T. Valimae, Zh. Org. Khim. 1984, 20(7), 1417-21, dort als "β-Campholennitril" bezeichnet).

Über olfaktorische Eigenschaften der beiden Campholenylnitrile **A** und **B** finden sich in der Literatur keine Angaben.

In der Geruchsbewertung hat das reine α-Campholennitril **A** einen stark ausstrahlenden, fruchtigen und süß-würzigen Duft in Richtung Tonka-Extrakt und Agrumenschalen mit Aspekten von Iriswurzel-Extrakt, während das reine γ-Campholennitril **B** einen stark ausstrahlenden Duft besitzt, der würzig und frisch-holzig wirkt und eine Nebennote in Richtung Cumin aufweist. Beide Campholennitrile verstärken Kopfnoten und Ausstrahlung von Parfümölen.

Die Geruchseffekte der Campholennitrile **A** und **B** sind originell und neuartig, und zwar sowohl in bezug auf die reinen Komponenten als auch in bezug auf Gemische daraus. Außerdem sind diese Geruchseffekte auch überraschend, sie unterscheiden sich einerseits deutlich von dem olfaktorischen Verhalten der bekannten Campholenderivate und sie waren andrerseits auch nicht aus den Riechstoff-Eigenschaften bekannter anderer Nitrile vorhersehbar, wobei hinzukommt, daß die relativ starken metallisch-fettigen Nebennoten, die für bekannte aliphatische Nitrile wie Decylnitril, Tridecen-2-nitril, Citronellylnitril oder Geranylnitril mit Citrus-Hauptnoten typisch, aber unerwünscht sind, bei den Nitrilen **A** und **B** nicht auftreten. Mithin bestätigt sich auch hier die allgemeine Erfahrung, daß die olfaktorischen Eigenschaften bekannter Riechstoffe keine zwingenden Rückschlüsse auf die Eigenschaften strukturverwandter Verbindungen zulassen, weil weder der Mechanismus der Duftwahrnehmung noch der Einfluß der chemischen Struktur auf die Duftwahrnehmung hinreichend erforscht sind, somit also normalerweise nicht vorhergesehen werden kann, ob ein abgeänderter Aufbau bekannter Riechstoffe überhaupt zur Änderung der olfaktorischen Eigenschaften führt und ob diese Änderungen positiv oder negativ beurteilt werden.

Zur Herstellung der Nitrile **A** und **B** kann in unterschiedlicher Weise vorgegangen werden, wobei sich zwei alternative Reaktionswege als besonders zweckmäßig erwiesen haben, nämlich:

1. Oximierung von Campholenaldehyd **2** und anschließende Dehydratisierung gemäß nachfolgendem Schema **I**:

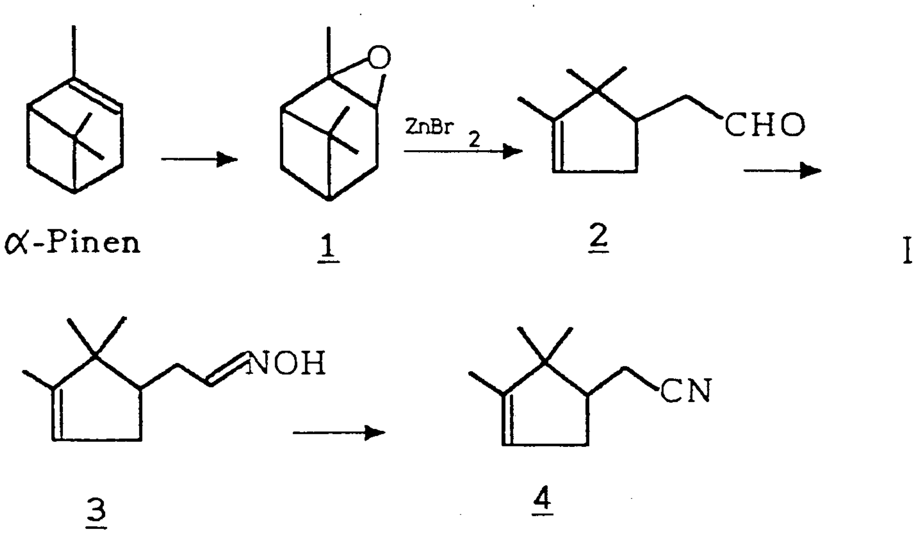


α-Pinen 1 2 I

3 4

Ausgehend von α-Campholenaldehyd **2**, dem Umlagerungsprodukt von α-Pinenepoxid, wurde das entsprechende Oxim **3** nach üblichen Methoden dargestellt und mit Acetanhydrid zum reinen α-Campholennitril **4** (**A**) dehydratisiert.

2. Umlagerung von Campheroxim **5** gemäß nachfolgendem Schema **II**.

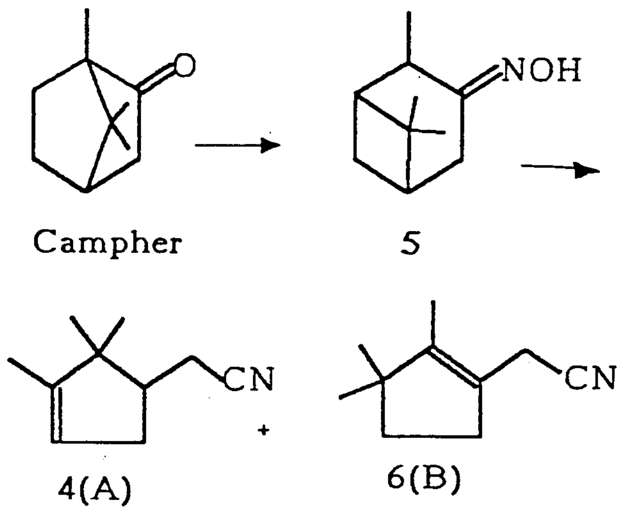

Campher 5

4(A) + 6(B)

II

Das aus Campher nach üblichen Methoden dargestellte Oxim **5** wurde nach der von Tiemann gegebenen Vorschrift mit verdünnter Schwefelsäure umgesetzt. Dabei ergab sich zunächst ein Gemisch von α-Campholennitril **4** und γ-Campholennitril **6**. Durch mehrstündiges Einwirken von Schwefelsäure wurde eine praktisch vollständige Umwandlung von α-Campholennitril in γ-Campholennitril erzielt. Somit lag erstmals reines γ-Campholennitril **6** (**B**) vor.

Unabhängig von dem eingeschlagenen Reaktionsweg lassen sich beide Nitrile **A** und **B** wahlweise, ausgehend von optisch aktiven oder inaktiven Edukten, als optisch aktive oder inaktive Produkte erzeugen, und es können sowohl die reinen Stereoisomeren als auch deren Gemische sowie Racemate erfindungsgemäß verwendet werden.

Die nachfolgenden Beispiele sollen die Erfindung erläutern, ohne sie einzuschränken.

**BEISPIEL 1**

| Parfümöl mit süß-krautiger Note | | |
|---|---|---|
| | a | b |
| Orangenöl | 180 g | 180 g |
| Isobornylacetat | 300 g | 300 g |
| Cyclogalbanat | 5 g | 5 g |
| Bergamottöl | 100 g | 100 g |
| Rosmarinöl, tunesisch | 30 g | 30 g |
| Beifußöl | 10 g | 10 g |
| Base Bigaradia 18*) | 15 g | 15 g |
| Apfelbase*) | 10 g | 10 g |
| Dimethyl-tetrahydrobenzaldehyd | 5 g | 5 g |
| Styrolylacetat | 20 g | 20 g |
| Hexylzimtaldehyd, alpha | 100 g | 100 g |
| Geraniumöl Bourbon, synth. | 10 g | 10 g |
| Phenyläthylalkohol | 30 g | 30 g |
| Citronellol | 100 g | 100 g |
| Ylanat (p-tert.-Butylcyclohexylacetat) | 30 g | 30 g |
| Lignofix*) (Acetylcedren) | 30 g | 30 g |
| Patchouliöl | 20 g | 20 g |
| Stemone (Givaudan) | 5 g | 5 g |
| Galbanum Resinoid | 10 g | 10 g |
| Moschus Ambrette | 20 g | 20 g |
| Cyclopentadecanolid | 20 g | 20 g |
| α-Campholennitril | - | 30 g |
| Dipropylenglycol | 30 g | - |
| | 1080 g | 1080 g |

*) Spezialprodukt DRAGOCO

Das Parfümöl a besitzt eine süß-krautige Note.

Die Komposition b, die anstelle des geruchsneutralen Dipropylenglycols ca. 3 % α-Campholennitril enthält, riecht kräftiger, frischer und zeigt eine interessante Betonung fruchtiger Aspekte. Sie ist sehr gut für Schaumbäder geeignet.

**BEISPIEL 2**

| Parfümöl mit würzig-holzigem charakter | | |
|---|---|---|
| | a | b |
| Evernyl ® | 5 g | 5 g |
| Brahmanol ® (DRAGOCO) | 5 g | 5 g |
| Eugenolmethylether | 5 g | 5 g |
| Decatone ® (Givaudan) 10% in DPG | 5 g | 5 g |
| Galaxolide ® (IFF) | 20 g | 20 g |
| Muskatnußöl | 20 g | 20 g |
| Lilial ® | 30 g | 30 g |
| Sauge Claree Resinoid | 35 g | 35 g |
| Eichenmoos Extrakt, grün, jugoslawisch (50 % in DPG) | 50 g | 50 g |
| Isobutylchinolin (10 % in DPG) | 50 g | 50 g |
| Moschus Keton | 60 g | 60 g |
| Eugenol | 80 g | 80 g |
| Linalylacetat | 100 g | 100 g |
| Lavandinöl | 100 g | 100 g |
| Bergamottöl (furocumarinfrei) | 100 g | 100 g |
| Lignofix (DRAGOCO) | 290 g | 290 g |
| Dipropylenglycol | 45 g | - |
| $\gamma$-Campholennitril | - | 45 g |
| | 1000 g | 1000 g |

Die Komposition a besitzt einen kräftigen, würzig-holzigen Duft, während bei Zugabe von $\gamma$-Campholennitril Komposition b eine sehr gewünschte Betonung süß-krautiger Noten (in Richtung Lavendel) sowie eine deutliche Abrundung festzustellen ist.

**BEISPIEL 3**

| Parfümöl mitherber Note | | |
|---|---|---|
| | a | b |
| Bergamottöl | 100 g | 100 g |
| Cedernblätteröl | 150 g | 150 g |
| Wermutöl | 30 g | 30 g |
| Rosmarinöl, tunesisch | 45 g | 45 g |
| Spiköl, spanisch | 20 g | 20 g |
| Muskatellersalbeiöl | 10 g | 10 g |
| Pfefferminzöl, brasilianisch | 5 g | 5 g |
| Phenyläthylalkohol | 100 g | 100 g |
| Citronellol | 30 g | 30 g |
| Diphenyläther | 10 g | 10 g |
| Geraniumöl Bourbon | 5 g | 5 g |
| Linalool | 50 g | 50 g |
| Linalylacetet | 60 g | 60 g |
| Methyljonon, gamma | 50 g | 50 g |
| Ylanat (4-tert.-Butylcyclohexylacetat) | 50 g | 50 g |
| Terpineol, rein | 100 g | 100 g |
| Anisaldehyd | 50 g | 50 g |
| Lignofix*) (Acetylcedrene) | 100 g | 100 g |
| Benzylsalicylat | 50 g | 50 g |
| Amylsalicylat | 70 g | 70 g |
| Ylang-Ylang-Öl, synth. | 30 g | 30 g |
| Galbanum-Extrakt | 5 g | 5 g |
| Cumarin | 50 g | 50 g |
| Cyclopentadecanolid | 30 g | 30 g |
| Dipropylenglycol | 40 g | - |
| $\gamma$-Campholennitril | - | 40 g |
| | 1240 g | 1240 g |

*) Spezialprodukt DRAGOCO

Das Parfümöl a besitzt einen herb-krautigen Duftkomplex.

Die Komposition b, die ca. 3 % $\gamma$-Campholennitril enthält, wirkt stärker, betont die krautige Note und zeichnet sich durch einen neuaufgetretenen Tee-Aspekt aus.

**BEISPIEL 4**

| Parfümöl blumigen Typs | | |
|---|---|---|
| | a | b |
| Brahmanol ® | 20 g | 20 g |
| Citronellol | 50 g | 50 g |
| Phenyläthylalkohol | 175 g | 175 g |
| Indol | 4 g | 4 g |
| Isoeugenol | 6 g | 6 g |
| Benzylacetat | 30 g | 30 g |
| Linalool | 40 g | 40 g |
| Terpineol, rein | 240 g | 240 g |
| Muguetalkohol* (2,2-Dimethyl-3-phenyl-propanol) | 200 g | 200 g |
| Zimtalkohol | 40 g | 40 g |
| Citronellylacetat | 20 g | 20 g |
| Frambinon* | 15 g | 15 g |
| Buccoxime* (1,5-Dimethyl-8-hydroximino-bicyclo 3.2.1 octan) | 70 g | 70 g |
| Dipropylenglycol | 90 g | 87 g |
| α-Campholennitril (A) | - | 2 g |
| γ-Campholennitril (B) | - | 1 g |
| | 1000 g | 1000 g |

*) Spezialprodukt der DRAGOCO

Die Komposition a besitzt eine kräftige, weich-blumige Note in Richtung Maiglöckchen mit exotisch-fruchtiger Nebennote. Die Zugabe geringer Mengen der Verbindungen **A** und **B** (Komposition b) ergibt eine andere Geruchscharakteristik in der interessanten Richtung "weiße Blüten" mit "Chypre"-Aspekten.

**BEISPIEL 5**

Die nachfolgende Tabelle veranschaulicht die außerordentlich hohe Stabilität des α-Campholennitrils in verschiedenen Grundmassen (die für das γ-Campholennitril in analoger Weise gilt). Von den vorgesehenen drei Bewertungsnoten für die Stabilität tritt überwiegend "Sehr Gut" auf, es ist nur einmal "Gut" und kein einziges Mal "Nicht Gut" gefunden worden.

| Testbase | Dosierung (%) | Geruchsstabilität 1 Monat Raumtemp. | 1 Monat 40°C | 3 Monate Raumtemp. | 3 Monate 40°C | Geruchseffekt (Überdeckung der Grundmasse, Ausstrahlung) |
|---|---|---|---|---|---|---|
| Saurer Reiniger ($H_3PO_4$) | 0,5 | SG | SG | SG | G | stark |
| Alkalischer Reiniger | 0,5 | SG | SG | SG | SG | stark |
| Seife, weiß | 1,0 | SG | SG | SG | SG | stark |
| Vollwaschmittel mit TAED | 0,2 | SG | SG | SG | SG | sehr stark |
| Vollwaschmittel flüssig | 0,3 | SG | SG | SG | SG | stark |
| Wäscheweich | 0,2 | SG | SG | SG | SG | sehr stark |
| Schaumbad | 3,0 | SG | SG | SG | SG | sehr stark |
| Shampoo | 0,5 | SG | SG | SG | SG | sehr stark |
| Aerosol-Antiperspirant | 4,0 | SG | SG | SG | SG | stark |
| Deo-Spray | 1,0 | SG | SG | SG | SG | stark |
| Tagescreme Öl-/Wasser-Emulsion | 0,3 | SG | SG | SG | SG | sehr stark |

Stabilität: SG = sehr gut (keine Änderung der Note)
G = gut (geringe Änderung der Note, akzeptabel)
NG = nicht gut (Note geändert, nicht akzeptabel)

## Patentansprüche

1. Verwendung von α- oder γ-Campholennitril (der Formel **A**, bzw. **B**) in reiner Form oder als Riechstoffe oder Bestandteile von Riechstoffmischungen zur Parfümierung von kosmetischen oder technischen

Verbrauchsgütern.

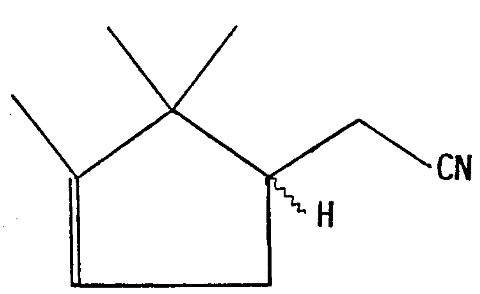

A

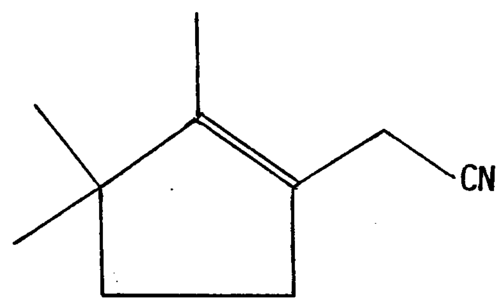

B

2. Verwendung von α-Campholenylnitril (**A**) als Riechstoff bzw. Bestandteil von Riechstoffmischungen zur Parfümierung kosmetischer oder technischer Verbrauchsgüter.

3. Verwendung von γ-Campholenylnitril (**B**) als Riechstoff bzw. Bestandteil von Riechstoffmischungen zur Parfümierung kosmetischer oder technischer Verbrauchsgüter.

4. Verwendung von Gemischen aus α-Campholenylnitril (**A**) und γ-Campholenylnitril (**B**), gekennzeichnet durch einen Gehalt von 0,5 - 99,5 % α-Campholenylnitril (**A**) bzw. 99,5 - 0,5 % γ-Campholenylnitril (**B**), als Riechstoffe bzw. Bestandteile von Riechstoffmischungen zur Parfümierung kosmetischer oder technischer Verbrauchsgüter.

**Claims**

1. Use of alpha or gamma campholene nitrile (of formula A or B) in pure form either as odorants or as ingredients of odorant mixtures for perfuming cosmetics or technical consumer goods.

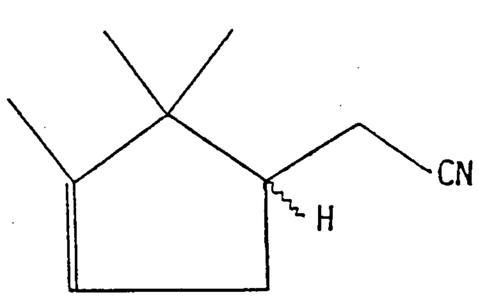

A

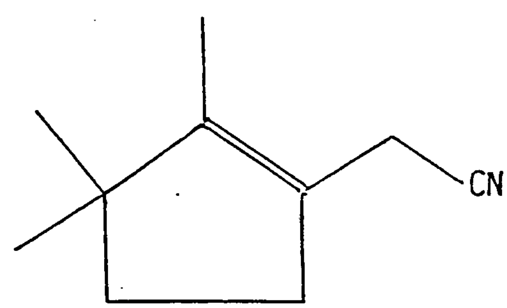

B

2. Use of alpha campholene nitrile (A) as odorant or as an ingredient of odorant mixtures for perfuming of cosmetic or technical consumer goods.

3. Use of gamma campholene nitrite (B) as odorant or as an ingredient of odorant mixtures for perfuming cosmetic or technical consumer goods.

4. Use of mixtures of alpha-campholene nitrile (A) and gamma-campholene nitrile (B), characterised by a content of 0.5 to 99.5% alpha-campholene nitrile (A) and 99.5% to 0.5% gamma-campholene nitrile (B), as odorants or as ingredients of odorant mixtures for perfuming of cosmetic or technical consumer goods.

## Revendications

1. Utilisation de α-campholènnitrile ou de γ-campholènnitrile (Formule A ou B) sous forme pure ou comme parfum ou constituant de mélanges parfumés pour parfumer des produits de consommation cosmétiques ou techniques.

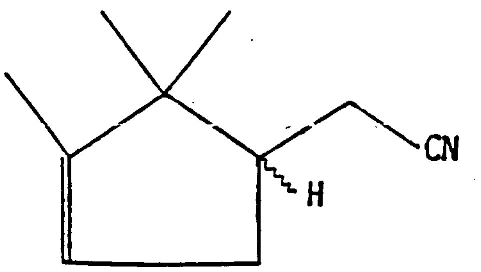

A

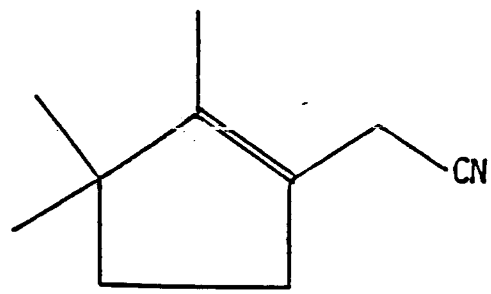

B

2. Utilisation de α-campholènylnitrile (A) comme parfum ou constituant de mélanges parfumés pour parfumer des produits de consommation cosmétiques ou techniques.

3. Utilisation de γ-campholènylnitrile (B) comme parfum ou constituant de mélanges parfumés pour parfumer des produits de consommation cosmétiques ou techniques.

4. Utilisation de mélanges de α-campholènylnitrile (A) et de γ-campholènylnitrile (B), caractérisée par une teneur de 0,5 à 99,5 % d'α-campholènylnitrile (A ou 99,5 à 0,5 % de γ-campholènylnitrile (B) comme parfum ou constituant de mélanges parfumés pour parfumer des produits de consommation cosmétiques ou techniques.